# EUROPEAN PATENT SPECIFICATION

(11) **EP 3 562 797 B1**
(45) Date of publication and mention of the grant of the patent: **13.10.2021**
(21) Application number: 17908720.0
(22) Date of filing: 08.12.2017
(51) Int. Cl.: C06B 25/04, C07D 233/92, C06B 25/34

(54) **NEW GENERATION PRIMARY EXPLOSIVE**
NEUE GENERATION VON PRIMÄRSPRENGSTOFFEN
EXPLOSIF PRIMAIRE DE NOUVELLE GÉNÉRATION

(30) Priority: 28.12.2016 TR 201619894
(43) Date of publication of application: 06.11.2019
(73) Proprietor: Atilim Universitesi, Golbasi/Ankara (TR)
(72) Inventor: ÖZALP YAMAN, Seniz, Gölbasi/Ankara (TR); GERÇEK, Zuhal, Zonguldak (TR)
(74) Representative: Sevinç, Cenk
(86) International application number: PCT/TR2017/050639
(87) International publication number: WO 2018/203863

(56) References cited:
- WO-A1-2010/021409
- WO-A2-2008/048351
- US-A- 4 028 154
- J. W. SUWINSKI: "1,4-Dinitro-1H-imidazoles", Reviews and Accounts , ARKIVOC, vol. 2015, no. i 30 January 2015 (2015-01-30), pages 97-135, XP055563415, Retrieved from the Internet: URL:http://dx.doi.org/10.3998/ark.5550190. p008.815
- K. D. OYLER et al.: "Green Explosives: Potential Replacements For Lead Azide And Other Toxic Detonator And Primer Constituents", U.S. Army RDECOM-ARDEC, 2 February 2016 (2016-02-02), XP055563421,
- Lemi Turker: "Azo-bridged triazoles: Green energetic materials", Defence Technology, vol. 12 13 November 2015 (2015-11-13), pages 1-15, XP055563426, Retrieved from the Internet: URL:https://doi.org/10.1016/j.dt.2015.11.0 02

## Description

### Technical Field

The invention relates to a new generation primary explosive that can be used in lieu of lead styphnate and/or lead azide particularly at the defense industry, that doesn't cause any environmental pollution, and that shall have no negative impact on the human health, and the synthesis method thereof.

### State of Art

Nowadays, the defense requirements of the countries are of paramount importance, and majority of the research, development efforts and investments are made on the defense industry. The endeavors of the countries intended for diminishing their dependence to the foreign countries with respect to defense are of paramount importance. This fact is also applicable for our country, and production or development of primary explosives used at commerce and defense industry by our own resources and methods is very important with respect to homeland security and financial independence.

The explosion at the primary explosive materials, which causes significant destruction and which can be detonated easily by means of detonation mechanism or impact, occurs as a result of chemical reaction, which reveals an extremely high energy. Physical/chemical aspects such as transportability, sensitivity, durability, degradation effect and toxicity, etc. as well as the economic aspects such as production costs, etc. are very important for the explosive substances that also find use in the everyday life apart from the defense industry, such as applicability for extraction of ores. Examples for the substances that can be used as primary explosives include lead azide or lead styphnate and substances with lead content that present chemical or thermal characteristics similar to such compounds. However, NATO decided to terminate use of such explosives with lead content that are extremely hazardous and detrimental for human health and environment in the long term, which, in turn, necessitated production of alternate primary explosives.

In the state of art, application no. US2013204005 mentions about the compounds which doesn't contain lead and can be used as primary explosive, and discloses copper(I) nitrotetrazolate compound and the synthesis method thereof.

Application no. US9440934, on the other hand, mentions about copper(I) 5-nitrotetrazolate compound obtained from 5-aminotetrazolate (5-AT), which can be used as primary explosive, and the synthesis method thereof.

In the state of art, Suwinski, J. W. has comprehensively reviewed the synthesis, properties and reactions of 1,4-dinitro-1H-imidazoles as well as the hazards associated with working with them in an article "1,4-Dinitro-1H-imidazoles". In the subject document, it is also stated that 1,4-dinitro-1H-imidazole is the most convenient starting compound and many imidazole derivatives substituted with nitro and methyl groups and are used as explosives. However, there is no indication how this explosive property is maintained with these imidazole derivatives subsituted nitro, methyl groups and interrelatedly how to provide alternative primary explosive compounds which are preferred in defense industry in lieu of lead styphnate and/or lead azide in order not to cause any environmental pollution and negative impact on human health [1].

Oyler *et al.* has investigated the replacements for lead-based explosives such as lead azide (LA) and lead styphnate (LS) and focusing on high nitrogen molecules as well as inorganic nanoporous silicon/oxidizer composites in an article "GREEN EXPLOSIVES: POTENTIAL REPLACEMENTS FOR LEAD AZIDE AND OTHER TOXIC DETONATOR AND PRIMER CONSTITUENTS". However, the subject document offers solution for green replacement candidates, with a focus on application in stab detonators such as the M55 and does not provide a solution to the replacement for LA by using 1,4-dinitro-1H-imidazoles and derivatives [2].

Türker, L. has focused on the most recent investigations by excerpting from the literature of azo-bridged triazoles (mainly 1,2,4-triazoles), some of their derivatives (chloromethyl, dinitro and trinitro pyrazole substituted ones, etc.) and some of their salts in an article "Azo-bridged triazoles: Green energetic materials" [3].

In the other state of art, patent application no. WO2010021409 (A1) relates to a method for the production of 2-halo-4-nitroimidazole and intermediates thereof.

Patent application no. WO2008048351 (A2) provides the compound copper (I) nitrotetrazolate. Certain embodiments of this prior art document provide methods for preparing lead- free primary explosives. The method includes: providing cuprous salt; providing water; providing 5 -nitrotetrazolate salt; combining the cuprous salt, water and 5- nitrotetrazolate salt to form a mixture; and heating the mixture. However, said applications not only fail to achieve the efficiency and chemical properties of the primary explosives to the full extent, but also fails to produce explosives with required potency and stability.

US4028154 discloses that ammonium 2,4,5-trinitroimidazole has an explosive performance comparable to RDX.

### The Problems that the Invention Aims to Solve

The objective of the invention is to develop new generation primary explosive compounds that can be used in lieu of lead styphnate and/or lead azide particularly at the defense industry, that doesn't cause any environmental pollution, and that shall have no negative impact on the human health, and the synthesis method thereof.

Another objective of the invention is to obtain compounds which can be used not only as primary explosives, but also as starting material for synthesis of many novel primary explosive compounds.

Yet another objective of the invention is to ensure elimination of foreign dependency in the defense industry through use of our own resources in production of the primary explosive.

### Description of the Invention

The invention is 4-nitro-5(dinitromethyl)imidazole, 4-nitro-5(dinitromethyl)imidazole sodium salt, 4-nitro-5(dinitromethyl)imidazole ammonium salt compounds, illustrated with formula I, II and III, which can be used as both the starting material for synthesis of many novel primary explosive compounds, and as primary explosive, that can be preferred in lieu of lead styphnate and/or lead azide particularly at the defense industry, that doesn't cause any environmental pollution, and that shall have no negative impact on the human health.

The primary explosive starting material of the invention is 4-nitro-5(dinitromethyl)imidazole obtained by addition of hydroxyl amine hydrochloride to 4-nitro-1H-imidazole-5-carbonitrile, it is characterized with formula I.

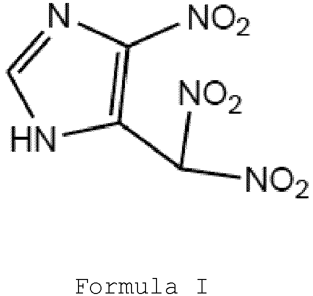

The primary explosive starting material of the invention is 4-nitro-5(dinitromethyl)imidazole sodium salt obtained by addition of sodium chloride (NaCl) to 4-nitro-5(dinitromethyl)imidazole ligand characterized with formula I, it is characterized with formula II.

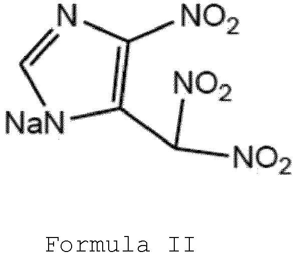

The primary explosive starting material of the invention is 4-nitro-5(dinitromethyl)imidazole ammonium salt obtained by addition of ammonium chloride (NH₄Cl) to 4-nitro-5(dinitromethyl)imidazole ligand characterized with formula I, it is characterized with formula III.

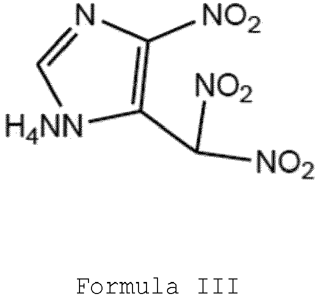

4-nitro-5(dinitromethyl)imidazole compound should be synthesized first in the synthesis process of 4-nitro-5(dinitromethyl)imidazole, 4-nitro-5(dinitromethyl)imidazole sodium salt and 4-nitro-5 (dinitromethyl) imidazole ammonium salt, which can be used as the primary explosive starting material of the invention. The synthesis of 4-nitro-5(dinitromethyl)imidazole compound is performed in three phases.

The synthesis method of 4-nitro-5(dinitromethyl)imidazole compound (Formula I);

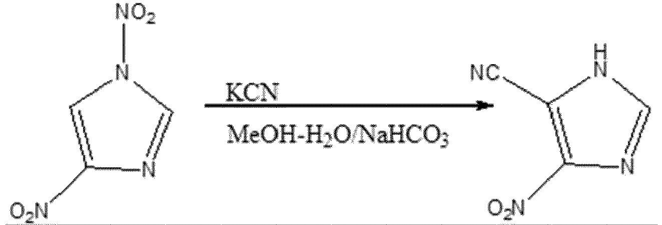

Phase I comprises the process steps set forth hereunder;
- Addition of 15-25 mmol of 4-nitro-1H-imidazole-5-carbonitrile to 40-60 ml of water and 20-35 ml of isopropanol mixture, and dissolution therein,
- Addition of anhydrous ethane carbonate at an amount equal to the solution at minimum,
- Heating the mixture to 40-60°C,
- Addition of 35-50 mmol of hydroxyl amine hydrochloride to the mixture and agitating the mixture for 1-3 hours,
- Washing the solution so obtained with ice ethane and drying under vacuum,
- Obtaining 4N'-hydroxy-4-nitro-1H-imidazole-5-carboxymidamide by recrystallization of the solid so obtained in ethanol.

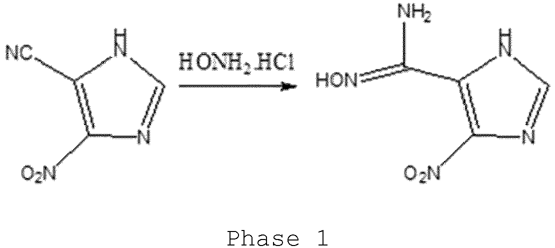

Phase II comprises the process steps set forth hereunder;
- Dissolving 10-20 mmol of N'-hydroxy-4-nitro-1H-imidazole-5-carboxymidamide in 40-60 ml of concentrated hydrochloric acid and 25-40 ml of water,
- Cooling the mixture to 0°C,
- Adding 10-20 ml of saturated sodium nitrite solution to the mixture dropwise and agitating for 1-3 hours,
- Heating the mixture to the ambient temperature and agitating for 1-3 hours for completing outgassing of N₂ gas from the solution,
- Obtaining N-hydroxy-4-nitro-1H-imidazole-5-carbimidoil chloride by washing the obtained precipitate with ice water and drying under vacuum.

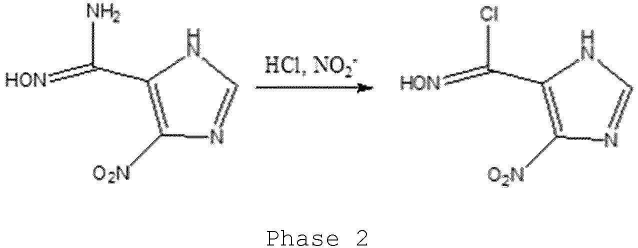

Phase III comprises the process steps set forth hereunder;
- Preparing N-hydroxy-4-nitro-1H-imidazole-5-carbimidoil chloride suspension in 40-55 ml of chloroform (CHCl₃),
- Addition of 15-25 mmol of N₂O₅ to obtained suspension,
- Agitating the mixture for 30-50 minutes at 40-55°C,
- Removing the solution with rotary evaporator under vacuum,
- Obtaining 4-nitro-5(dinitromethyl)imidazole compound by purifying the obtained product with ethyl acetate(EtOAc): Pentane solvent mixture by means of colon chromatography.

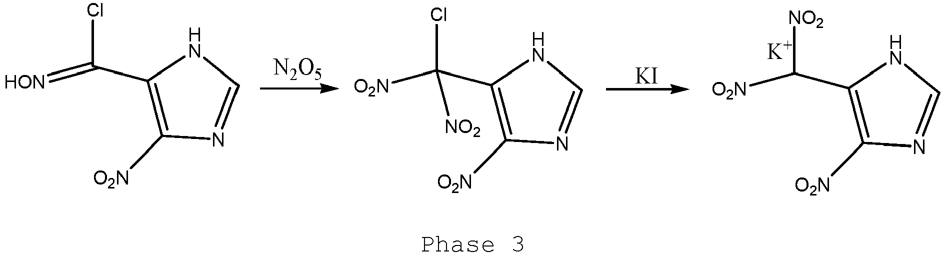

Synthesis of 4-nitro-5(dinitromethyl)imidazole sodium salt compound is performed by using 4-nitro-5(dinitromethyl)imidazole shown with Formula I.

The synthesis method of 4-nitro-5(dinitromethyl)imidazole sodium salt (formula II) comprises the process steps set forth hereunder;
- Dissolving 4-nitro-5(dinitromethyl)imidazole in dichloromethane solvent,
- Treating the solution with saturated sodium bicarbonate (NaHCO₃),
- Addition of saturated sodium chloride (NaCl) to the mixture,
- Filtering and removing white solid formed,
- Drying the product so obtained.

Synthesis of 4-nitro-5(dinitromethyl)imidazole ammonium salt compound is performed by using 4-nitro-5(dinitromethyl)imidazole shown with Formula I.

The synthesis method of 4-nitro-5(dinitromethyl)imidazole ammonium salt (formula III) comprises the process steps set forth hereunder;
- Dissolving 4-nitro-5(dinitromethyl)imidazole in dichloromethane solvent,
- Treating the solution with saturated NaHCO₃,
- Addition of saturated ammonium chloride (NH₄Cl) to the mixture,
- Filtering and removing white solid formed,
- Drying the product so obtained.

The compounds of the invention so obtained are free of toxic metals and heavy transition metals such as lead, mercury, silver, antimony, etc., thus do not induce environmental pollution. Furthermore, the compounds of the invention do not contain any wastes that are detrimental to the human health.

### Method of Implementation of the Invention to the Industry:

The new generation primary explosive of the invention and the synthesis method thereof that can be used in lieu of lead styphnate and/or lead azide particularly at the defense industry, that doesn't cause any environmental pollution, and that shall have no negative impact on the human health, and, is capable of presenting superior characteristics than the primary explosives as they can also be used as starting material for synthesis of many novel primary explosive compounds.

### REFERENCES

1. Suwinski, J. W. (2015). 1,4-Dinitro-1H-imidazoles.
2. Oyler, K., Mehta, N., Cheng, G., & Redner, P. (n.d.). GREEN EXPLOSIVES: POTENTIAL REPLACEMENTS FOR LEAD AZIDE AND OTHER TOXIC DETONATOR AND PRIMER CONSTITUENTS.
3. Türker, L. (2016). Azo-bridged triazoles: Green energetic materials. Defence Technology, 12(1).

## Claims

1. Compounds that can be used as primary explosive **characterized in that**, said compounds are;
• 4-nitro-5(dinitromethyl)imidazole obtained by addition of hydroxyl amine hydrochloride to 4-nitro-1H-imidazole-5-carbonitrile, shown with formula I; 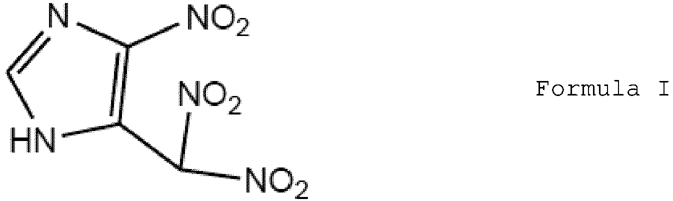 or
• 4-nitro-5(dinitromethyl)imidazole sodium salt, shown with formula II, obtained by addition of sodium chloride (NaCl) to 4-nitro-5(dinitromethyl)imidazole ligand, characterized with formula I; 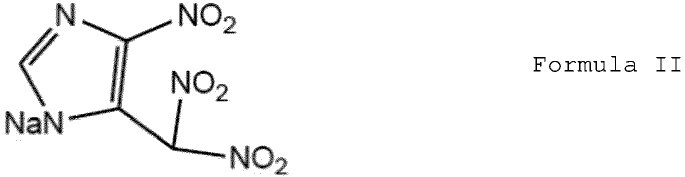 or
• 4-nitro-5(dinitromethyl)imidazole ammonium salt, shown with formula III, obtained by addition of ammonium chloride (NH₄Cl) to 4-nitro-5(dinitromethyl)imidazole ligand, characterized with formula I; 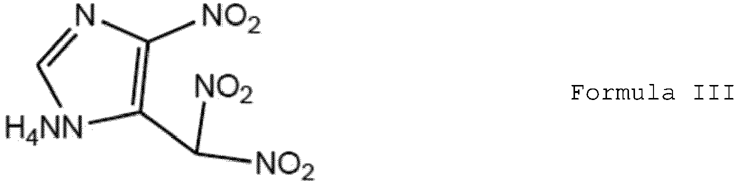 which can be used as both the starting material for synthesis of many novel primary explosive compounds, and as primary explosive, that can be preferred in lieu of lead styphnate and/or lead azide particularly at the defense industry, that doesn't cause any environmental pollution, and that shall have no negative impact on the human health.

2. A synthesis method of the compounds that can be used as primary explosive according to Claim 1, **characterized in that** the synthesis method of 4-nitro-5(dinitromethyl)imidazole compound shown with formula I comprises;
Phase I comprising process steps of:
• Addition of 15-25 mmol of 4-nitro-1H-imidazole-5-carbonitrile to 40-60 ml of water and 20-35 ml of isopropanol mixture, and dissolution therein,
• Addition of anhydrous ethane carbonate at an amount equal to the solution at minimum,
• Heating the mixture to 40-60°C,
• Addition of 35-50 mmol of hydroxyl amine hydrochloride to the mixture and agitating the mixture for 1-3 hours,
• Washing the solution so obtained with ice ethane and drying under vacuum,
• Obtaining 4N'-hydroxy-4-nitro-1H-imidazole-5-carboxymidamide by recrystallization of the solid so obtained in ethanol,
Phase II comprising process steps of:
• Dissolving 10-20 mmol of N'-hydroxy-4-nitro-1H-imidazole-5-carboxymidamide in 40-60 ml of concentrated hydrochloric acid and 25-40 ml of water,
• Cooling the mixture to 0°C,
• Adding 10-20 ml of saturated sodium nitrite solution to the mixture dropwise and agitating for 1-3 hours,
• Heating the mixture to the ambient temperature and agitating for 1-3 hours for completing outgassing of N₂ gas from the solution,
• Obtaining N-hydroxy-4-nitro-1H-imidazole-5-carbimidoil chloride by washing the obtained precipitate with ice water and drying under vacuum, and
Phase III comprising process steps of
• Preparing N-hydroxy-4-nitro-1H-imidazole-5-carbimidoil chloride suspension in 40-55 ml of chloroform (CHCl₃),
• Addition of 15-25 mmol of N₂O₅ to obtained suspension,
• Agitating the mixture for 30-50 minutes at 40-55°C,
• Removing the solution with rotary evaporator under vacuum,
• Obtaining 4-nitro-5(dinitromethyl)imidazole compound by purifying the obtained product with ethyl acetate(EtOAc): Pentane solvent mixture by means of colon chromatography.

3. A synthesis method of the compounds that can be used as primary explosive according to Claim 2, **characterized in that**, the synthesis method of 4-nitro-5(dinitromethyl)imidazole sodium salt compound shown with formula II comprises process steps of;
• Dissolving 4-nitro-5(dinitromethyl)imidazole in dichloromethane solvent,
• Treating the solution with saturated sodium bicarbonate (NaHCO₃),
• Addition of saturated sodium chloride (NaCl) to the mixture,
• Filtering and removing white solid formed,
• Drying the product so obtained.

4. A synthesis method of the compounds that can be used as primary explosive according to Claim 2, **characterized in that**, the synthesis method of 4-nitro-5(dinitromethyl)imidazole ammonium salt shown with formula III comprises process steps of;
• Dissolving 4-nitro-5(dinitromethyl)imidazole in dichloromethane solvent,
• Treating the solution with saturated NaHCO₃,
• Addition of saturated ammonium chloride (NH₄Cl) to the mixture,
• Filtering and removing white solid formed,
• Drying the product so obtained.

## Patentansprüche

1. Verbindungen, die als primärer Explosivstoff verwendet werden können, **dadurch gekennzeichnet, dass** diese Verbindungen sind;
• 4-Nitro-5(dinitromethyl)imidazol, erhalten durch die Zugabe von Hydroxylamin-Hydrochlorid zum 4-Nitro-1H-imidazol-5-carbonitril, angegeben mit Formel I; 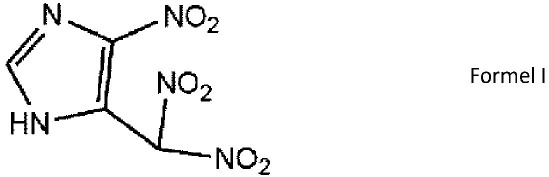 oder
• 4Nitro-5(dinitromethyl)imidazol-Natriumsalz, angegeben mit Formel II, erhalten durch die Zugabe von Natriumchlorid (NaCl) zum 4-Nitro-5(dinitromethyl)imidazol-Liganden, gekennzeichnet mit Formel I; 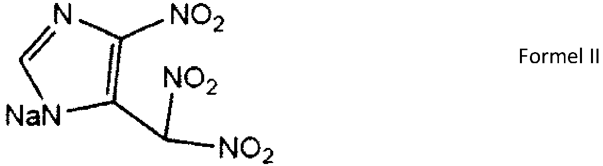 oder
• 4-Nitro-5(dinitromethyl)imidazol-Ammoniumsalz, angegeben mit Formel III, erhalten durch die Zugabe von Ammoniumchlorid (NH₄Cl) zum 4-Nitro-5(dinitromethyl)imidazol-Liganden, angegeben mit Formel I; 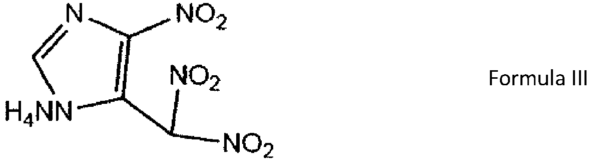 das sowohl als Ausgangsmaterial für die Synthetisierung von vielen neuen primären Explosivstoffen verwendet werden kann, als auch als primärer Explosivstoff, der anstelle von Bleistyphnat und/oder Bleiazid, insbesondere in der Verteidigungsindustrie bevorzugt werden kann, der keine Umweltverschmutzung zur Folge hat und der keine negativen Auswirkungen auf die menschliche Gesundheit haben soll.

2. Syntheseverfahren für die Verbindungen, die als primärer Explosivstoff verwendet werden können, nach Anspruch 1, **dadurch gekennzeichnet, dass** das Syntheseverfahren der 4-Nitro-5(dinitromethyl)imidazol-Verbindung angegeben mit Formel I umfasst;
Die Stufe I mit den folgenden Verfahrensschritte:
• Zugabe von 15-25 mmol 4-Nitro-1H-imidazol-5-carbonitril zu 40-60 ml Wasser und 20-35 ml einer Isopropanol-Mischung und Auflösen darin,
• Zugabe von anhydrischem Ethan-Karbonat in einer Menge, die der Lösung im Minimum gleicht,
• Erhitzen der Mischung auf 40-60°C,
• Zugabe von 35-50 mmol Hydroxylamin-Hydrochlorid zur Mischung und Schütteln der Mischung für 1-3 Stunden,
• Waschen der so erhaltenen Lösung mit Eis-Ethan und Trocknen unter Vakuum,
• Erhalten von 4N'-Hydroxy-4-nitro-1H-imidazol-5-carboxymidamid durch Umkristallisieren des so erhaltenen Feststoffs in Ethanol,
Die Stufe II mit den folgenden Verfahrensschritte:
• Auflösen von 10-20 mmol N'-Hydroxy-4-nitro-1H-imidazol-5-carboxymidamid in 40-60 ml konzentrierter Salzsäure und 25-40 ml Wasser,
• Kühlen der Mischung auf 0°C,
• Zugabe von 10-20 ml gesättigter Natrium-Nitrit-Lösung zur Mischung tropfenweise und Schütteln für 1-3 Stunden,
• Erhitzen der Mischung auf Umgebungstemperatur und Schütteln für 1-3 Stunden, um das Ausgasen von N₂ aus der Lösung zu beenden,
• Erhalten von N-Hydroxy-4-nitro-1H-imidazol-5-carbimidoylchlorid durch Waschen des erhaltenen Niederschlags mit Eiswasser und Trocknen unter Vakuum und
Die Stufe III mit den folgenden Verfahrensschritte
• Bereitstellen einer Suspension von N-Hydroxy-4-nitro-1H-imidazol-5-carbimidoilchlorid in 40-55 ml Chloroform (CHCl₃),
• Zugabe von 15-25 mmol N₂O₅ zur erhaltenen Suspension,
• Schütteln der Mischung für 30-50 Minuten bei 40-55°C,
• Entfernen der Lösung mit einem Drehverdampfer unter Vakuum,
• Erhalten einer 4-Nitro-5(dinitromethyl)imidazol-Verbindung durch Reinigen des erhaltenen Produkts mit Ethylacetat (EtOAc): Pentan-Lösungsmittel-Mischung mittels Kolon-Chromatographie.

3. Syntheseverfahren für die Verbindungen, die als primärer Explosivstoff verwendet werden können, nach Anspruch 2, **dadurch gekennzeichnet, dass** das Syntheseverfahren der 4-Nitro-5(dinitromethyl) imidazol-Natriumsalz-Verbindung, angegeben mit Formel II die folgende Verfahrensschritte umfasst;
• Auflösen von 4-Nitro-5(dinitromethyl)imidazol in Dichlormethan-Lösungsmittel,
• Behandlung der Lösung mit gesättigtem Natriumbicarbonat (NaHCO₃),
• Zugabe von gesättigtem Natriumchlorid (NaCl) zu der Mischung,
• Filtrieren und Entfernen des gebildeten weißen Feststoffs,
• Trocknen des so erhaltenen Produkts.

4. Syntheseverfahren für die Verbindungen, die als primärer Explosivstoff verwendet werden können, nach Anspruch 2, **dadurch gekennzeichnet, dass** das Syntheseverfahren des 4-Nitro-5(dinitromethyl)imidazol-Ammoniumsalzes, angegeben mit Formel III die folgende Verfahrensschritte umfasst;
• Auflösen von 4-Nitro-5(dinitromethyl)imidazol in Dichlormethan-Lösungsmittel,
• Behandlung der Lösung mit gesättigtem NaHCO₃,
• Zugabe von gesättigtem Ammoniumchlorid (NH₄Cl) zu der Mischung,
• Filtrieren und Entfernen des gebildeten weißen Feststoffs,
• Trocknen des so erhaltenen Produkts.

## Revendications

1. Composés utilisables comme explosif primaire, **caractérisés en ce que** lesdits composés sont:
• le 4-nitro-5 (dinitrométhyle) imidazole obtenu par addition du chlorure d'hydroxylammonium au 4-nitro-1H-imidazole-5-carbonitrile, représenté par la formule I; 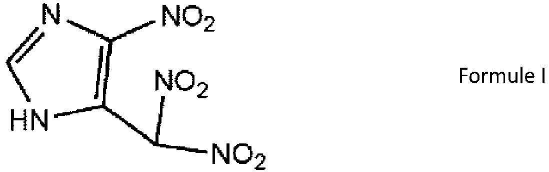 ou
• le sel de sodium du 4-nitro-5 (dinitrométhyle) imidazole, représenté par la formule II, obtenu par addition du chlorure de sodium (NaCl) au ligand de 4-nitro-5 (dinitrométhyle) imidazole **caractérisé par** la formule I; 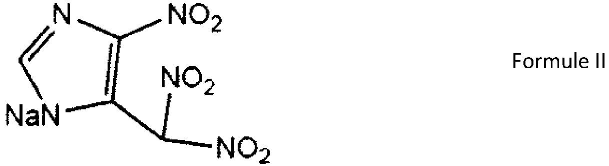 ou
• le sel d'ammonium du 4-nitro-5 (dinitrométhyle) imidazole, représenté par la formule III, obtenu par addition du chlorure d'ammonium (NH₄Cl) au ligand de 4-nitro-5 (dinitrométhyle) imidazole **caractérisé par** la formule I; 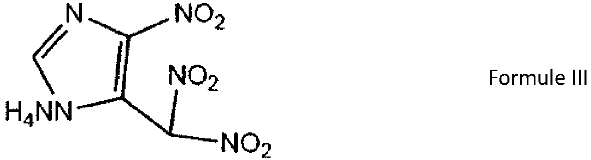 qui peut être utilisé à la fois comme la matière première pour la synthèse de nombreux nouveaux composés d'explosifs primaires et comme l'explosif primaire, qui peut être préféré au lieu du styphnate de plomb et/ou de l'azoture de plomb en particulier dans l'industrie de la défense, qui ne cause aucune pollution environnementale, et qui n'aura aucun impact négatif sur la santé humaine.

2. Une méthode de synthèse des composés utilisables comme explosif primaire selon la revendication 1, **caractérisé en ce que** la méthode de synthèse du composé 4-nitro-5 (dinitrométhyl) imidazole représenté par la formule I comprend;
Une phase I comprenant les étapes de procède suivantes:
• Ajouter 15-25 mmol du 4-nitro-1H-imidazole-5-carbonitrile dans 40-60 ml d'eau et 20-35 ml de la mélange d'isopropanol, et y dissoudre,
• Ajouter le carbonate d'éthane anhydre en quantité égale à la solution au minimum,
• Chauffer le mélange à 40-60°C,
• Ajouter 35 à 50 mmol de chlorhydrate d'hydroxylamine au mélange et agiter le mélange pendant 1-3 heures,
• Laver la solution ainsi obtenue avec la glace d'éthane et séchage sous vide,
• Obtenir 4N'-hydroxy-4-nitro-1H-imidazole-5-carboximidamide par recristallisation du solide ainsi obtenu dans l'éthanol,
Une phase II comprenant les étapes de procède suivantes:
• Dissoudre 10-20 mmol de N'-hydroxy-4-nitro-1H-imidazole-5-carboxymidamide dans 40-60 ml d'acide chlorhydrique concentré et 25-40 ml d'eau,
• Refroidir le mélange à 0°C,
• Ajouter 10-20 ml de la solution saturée du nitrite de sodium au mélange goutte à goutte et l'agiter pendant 1-3 heures,
• Chauffer le mélange à la température ambiante et l'agiter pendant 1-3 heures pour compléter le dégazage du gaz N₂ depuis la solution,
• Obtenir le chlorure de N-hydroxy-4-nitro-IH-imidazole-5-carbimidoil en lavant le précipité ainsi obtenu avec de l'eau glacée et le sécher sous vide, et
Une phase III comprenant les étapes de procède suivantes:
• Préparer une suspension de chlorure de N-hydroxy-4-nitro-IH-imidazole-5-carbimidoil dans 40-55 ml de chloroforme (CHCl₃),
• Ajouter 15-25 mmol de N₂O₅ à la suspension obtenue,
• Agiter le mélange pendant 30-50 minutes à 40-55°C,
• Eliminer la solution avec un évaporateur rotatif sous vide,
• Obtenir le composé du 4-nitro-5 (dinitrométhyle) imidazole en purifiant le produit obtenu avec un mélange de solvants d'acétate d'éthyle (EtOAc) : Pentane par la chromatographie sur colonne.

3. Une méthode de synthèse des composés utilisables comme explosif primaire selon la revendication 2, **caractérisé en ce que** la méthode de synthèse du composé du sel de sodium du 4-nitro-5 (dinitrométhyl) imidazole représenté par la formule II comprend les étapes de procède suivantes:
• Dissoudre 4-nitro-5 (dinitrométhyl) imidazole dans le solvant du dichlorométhane,
• Traiter la solution avec le bicarbonate de sodium saturé (NaHCO₃),
• Ajouter du chlorure de sodium saturé (NaCl) au mélange,
• Filtrer et éliminer le solide blanc formé,
• Sécher le produit ainsi obtenu.

4. Une méthode de synthèse des composés utilisables comme explosif primaire selon la revendication 2, **caractérisé en ce que** la méthode de synthèse du sel d'ammonium du 4-nitro-5 (dinitrométhyl) imidazole représenté par la formule III comprend les étapes de procède suivantes:
• Dissoudre 4-nitro-5 (dinitrométhyl) imidazole dans le solvant du dichlorométhane,
• Traiter la solution avec le NaHCO₃ saturé,
• Ajouter du chlorure d'ammonium saturé (NH₄Cl) au mélange,
• Filtrer et éliminer le solide blanc formé,
• Sécher le produit ainsi obtenu.
